(19) Europäisches Patentamt
European Patent Office
Office européen des brevets

(11) Publication number: 0 101 094 A1

(12) # EUROPEAN PATENT APPLICATION

(21) Application number: 83108089.0

(22) Date of filing: 16.08.83

(51) Int. Cl.$^3$: C 07 C 121/75
C 07 C 153/023, C 07 C 153/11
A 01 N 39/02

(30) Priority: 17.08.82 JP 142373/82

(43) Date of publication of application: 22.02.84 Bulletin 84/8

(84) Designated Contracting States: DE FR GB IT

(71) Applicant: Kanesho Co., Ltd.
No. 4-1, Marunouchi 2-chome Chiyoda-ku
Tokyo(JP)

(71) Applicant: TOYO SODA MANUFACTURING CO., LTD.
No. 4560, Ooaza Tonda Shinnanyo-shi
Yamaguchi-ken(JP)

(72) Inventor: Someya, Shinzo
20c-1108, Nakaarai, 3-chome
Tokorozawa-shi Saitama-ken(JP)

(72) Inventor: Akahira, Rokuro
13-14, Gakuen-machi, 1-chome
Higashikurume-shi Tokyo(JP)

(72) Inventor: Nonaka, Yuji
635-2, Ohaza Tonda
Shinnanyou-shi Yamaguchi-ken(JP)

(72) Inventor: Ito, Mikio
484-2, Ohaza Shimogami
Tokuyama-shi Yamaguchi-ken(JP)

(74) Representative: Vossius Vossius Tauchner Heunemann Rauh
Siebertstrasse 4 P.O. Box 86 07 67
D-8000 München 86(DE)

(54) **Cyanophenoxy-phenoxypentenoic acid derivative, process for the preparation thereof and herbicide composition containing the same.**

(57) A cyanophenoxy-phenoxypentenoic acid derivative which is excellent in selective herbicide function to kill weeds of Gramineae family and harmless to cereal plants. The cyanophenoxy-phenoxypentenoic acid derivative is represented by the general formula of:

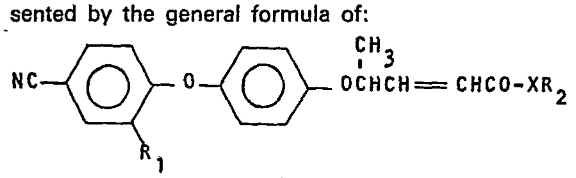
(I)

wherein $R_1$ is hydrogen, chlorine or bromine atom, X is oxygen or sulfur atom, and $R_2$ is hydrogen atom, cation, alkyl, fluoroalkyl, benzyl or $-N=C(dialkyl)$ group.
The cyanophenoxy-phenoxypentenoic acid is prepared by reacting, in the presence of a base, a cyanophenoxy-phenol derivative represented by the general formula of:

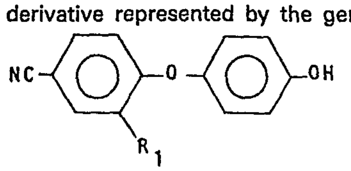
...........(II)

wherein $R_1$ is the same as set forth above;
with a 4-halocarboxylic acid derivative represented by the general formula of:

$$Hal\,\underset{\displaystyle |}{\overset{CH_3}{}}\!CH - A - COXR_2 \qquad \text{..... (III)}$$

wherein A is

$$-\underset{\underset{Hal}{|}}{CH}-CH_2$$

or $-CH=CH-$, Hal is a halogen atom and X and $R_2$ is the same as set forth above.

EP 0 101 094 A1

Our Ref.: S 588EP Augus 0101094

Case: OP 83227

KANESHO CO., LTD. and TOYO SODA MANUFACTURING CO., LTD.
Tokyo / Japan Shinnanyou-shi, Yamaguchi-ken / Japan

# Cyanophenoxy-phenoxypentenoic Acid Derivative, Process for the Preparation thereof and Herbicide Composition Containing the Same

## BACKGROUND OF THE INVENTION:

The present invention relates to a novel compound which has selective herbicide function, a process for the preparation thereof and a herbicide composition containing the same. More particularly, it relates to a cyanophenoxy-phenoxypentenoic acid derivative which is excellent in selective herbicide function to kill weeds of Gramineae family.

We have synthesized and investigated on a variety of pentenoic acid derivatives, and found that cyanophenoxy-phenoxypentenoic acid derivatives are excellent in herbicide function to kill weeds of Gramineae family with no appreciable harmful effect on cereal plants. The present invention is based on the above finding.

## SUMMARY OF THE INVENTION:

A primary object of the present invention is to provide a cyanophenoxy-phenoxypentenoic acid derivative which is excellent in selective herbicide function and represented by the following general formula of:

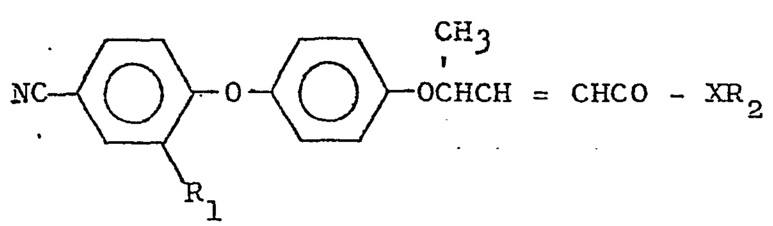 (I)

wherein $R_1$ is hydrogen, chlorine or bromine atom, X is oxygen or sulfur atom and $R_2$ is hydrogen atom, cation, alkyl, fluoroalkyl, benzyl or -N=C(dialkyl) group. The term "alkyl" throughout this specification refers to an alkyl group having 1 to 9, preferably 1 to 4 carbon atoms; the range of carbon atoms in the alkyl group of the fluoroalkyl group is preferably 1 to 3 and most preferably 1 to 2.

Another object of the invention is to provide a process for the preparation of the aforementioned cyanophenoxy-phenoxypentenoic acid derivative, wherein a cyanophenoxy-phenol derivative represented by the general formula of:

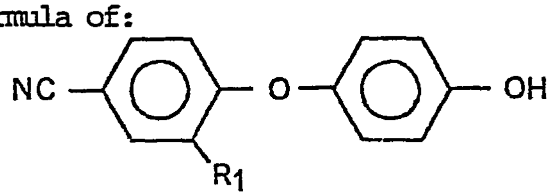 ······ (II)

wherein $R_1$ is hydrogen, chlorine or bromine atom; is reacted, in the presence of a base, with a 4-halocarboxylic acid derivative represented by the general formula of:

$$Hal\,\underset{}{\overset{CH_3}{\overset{|}{CH}}} - A - COXR_2 \quad \cdots\cdots\cdots\cdots \quad (III)$$

wherein A is $\underset{Hal}{\underset{|}{-CH}}-CH_2$ or -CH=CH-, Hal is a halogen atom, X is oxygen or sulfur atom, $R_2$ is hydrogen atom, cation, alkyl, fluoroalkyl, benzyl or -N=C(dialkyl) group.

A further object of this invention is to provide a herbicide composition containing the cyanophenoxy-phenoxypentenoic acid derivative.

DESCRIPTION OF THE INVENTION:

The cyanophenoxy-phenoxypentenoic acid derivative (hereinafter referred to as the compound of this invention) provided in accordance with the present invention, exerts superior selective herbicide function to kill weeds of Gramineae family, such as barnyard grass, crabgrass and foxtail (Setaria viridis Beaur), with no appreciable harmful effect on crop forbs, such as soybean, adzuki bean, beet or cotton, and also with no appreciable harmful effect on cereal plants, such as rice plant, wheat or barley.

When the compound of this invention is used for a foliage treatment, i.e. it is scattered over the stems and leaves of the plants growing on the field in an amount enough for killing the plants of Digitaria, Setaria, Echinochloa and Rottboellia families and Avena fatua L. completely, it does not adversely affect the growth of crop forbs including Japanese radish, Pisum sativum, Spinacia oleracea, adzuki bean, beet and cotton. When the compound of this invention is used to treat the soil in an amount enough for inhibiting germentation of barnyard grass, crabgrass and foxtail completely, it does not adversely affect the seeds of cereal plants including rice plant, wheat and barley. Accordingly, the compound of this invention may be used in an extremely wide application range.

The compound of this invention exerts strong herbicide function on barnyard grass which would bring a serious plague when rice plants are grown in a paddy field, with no adverse affect on the rice plants. Therefore, the compound of this invention may be used to kill

barnyard grass effectively in a paddy field where directly sown or transferred rice plants grow.

The compounds of this invention are prepared by the chemical reactions represented by the following reaction formulae, wherein Haℓ is a halogen atom, $R_1$ is hydrogen, chlorine or bromine atom, X is oxygen or sulfur atom, and $R_2$ is hydrogen atom, cation, alkyl, fluoroalkyl, benzyl or -N=C(dialkyl) group.

(1) 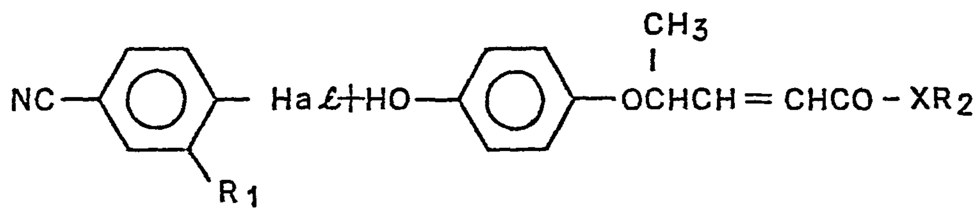

→ Compounds Represented by the General Formula [I]

(2) 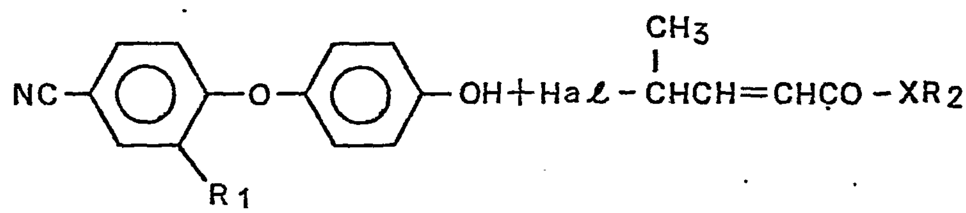

→ Compounds Represented by the General Formula [I]

(3) 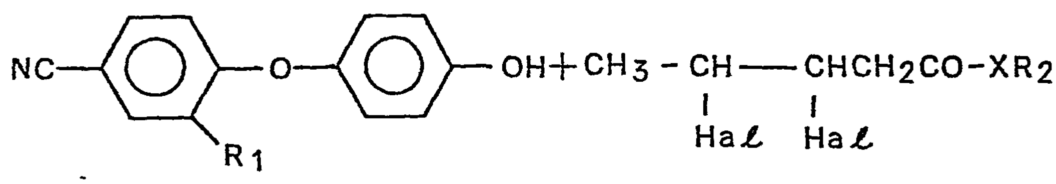

→ Compounds Represented by the General Formula [I]

(4) 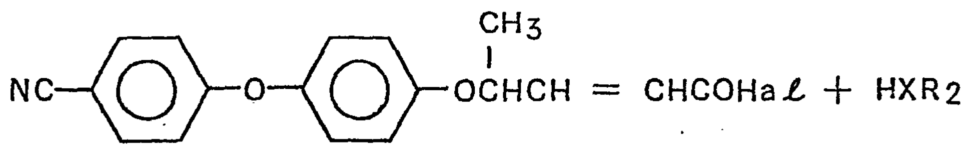

→ Compounds Represented by the General Formula [I]

As has been described hereinbefore, each of the reactions set forth above is carried out in the presence of a base.

If the $R_2$ of the second starting material is hydrogen atom, the resultant product may be converted to another derivative by esterification or neutralization.

On the other hand, if the $R_2$ of the second starting material is an ester, the ester group may be hydrolyzed so that the resultant product may be converted to a free carboxylic acid or a salt, or the ester group may be converted to another ester group by transesterification.

The reactions set forth above may be carried out in a solvent or a diluent which is inactive with the reactants. Preferable solvents are polar organic solvents, such as methyl ethyl ketone, dimethylformamide, dimethyl sulfoxide, alcohols and ketones. The reactions may be carried out at a temperature of 0°C to 200°C for 1 hour to several days. The used solvent and the reaction

temperature may be selected depending on the starting materials used. The reactions are carried out at a normal pressure. The bases which may be used in the aforementioned reactions as the condensating agents include the bases which have been commonly used for this purpose, the examples being KOH, $NaOCH_3$, $K_2CO_3$, potassium tert-butylate, triethylamine and pyridine.

The process for the preparation of the compound of this invention will now be described by referring to the synthesis examples thereof.

Synthesis Example 1: Preparation of Methyl 4- [4-(4-cyanophenoxy)phenoxy] -2-pentenoate (Compound No. 4)

30 ml of dimethyl sulfoxide was added with 0.56 g of potassium hydroxide and 2.22 g of methyl 4-(4-hydroxyphenoxy)-2-pentenoate, followed by stirring at room temperature for 30 minutes, and then further added with 1.37 g of 4-chlorobenzonitrile. After adding with additional 0.56 g of potassium hydroxide, the reaction liquid was heated at 80°C for 15 hours under agitation. After being cooled to room temperature, 1.70 g of methyl iodide was added to the reaction liquid and then the reaction liquid was stirred for 24 hours. Thereafter, the reaction liquid was poured into ice water, and then the reaction product was extracted with an ether. The ether phase was rinsed with a dilute hydrochloric acid solution and then with a saturated aqueous solution of sodium chloride, followed by drying the addition of anhydrous magnesium sulfate. Ether was distilled off to

obtain a residue which was refined by means of column chromatography (Carrier: Silica Gel, Deveoper: Benzene) to obtain 2.26 g (Yield: 70%) of an objective product which had an index of refraction of $n_D^{25}$ = 1.5682.

Synthesis Example 2: Preparation of Ethyl 4- [4(2-chloro-4-cyanophenoxy)phenoxy] -2-pentenoate (Compound No. 10)

30 ml of acetone was added with 2.10 g of 4-(2-chloro-4-cyanophenoxy)phenol, 1.43 g of potassium carbonate anhydride and 2.13 g of ethyl 4-bromo-2-pentenoate, and the reaction mixture was agitated for 8 hours under reflux. After cooling to room temperature, the inorganic compounds were filtered off and acetone was distilled off. The residue was refined by means of column chromatography (Carrier: Silica Gel, Developer: Benzene) to obtain 1.65 g of an objective product (Yield: 52%). The index of refraction of the product was $n_D^{25}$ = 1.5682.

Synthesis Example 3: Preparation of Ethyl 4- [4(2-bromo-4-cyanophenoxy)phenoxy] -2-pentenoate (Compound No. 11)

20 ml of ethanol was added with 1.45 g of 4-(2-bromo-4-cyanophenoxy)phenol, 1.10 g of potassium carbonate anhydride and 1.73 g of ethyl 3,4-dibromo-pentenoate, and agitated for 2 hours under reflux. After cooling to room temperature, and the reaction mixture was subjected to treating procedures similar to Synthesis Example 2 to obtain 1.33 g (Yield: 64%) of an objective product which had an index of refraction of $n_D^{25}$ = 1.5774.

Synthesis Example 4: Preparation of Isopropylideneamino 4- [4-(4-cyanophenoxy)-phenoxy]-2-pentenoate (Compound No.15)

Ethyl 4-[4-(4-cyanophenoxy)phenoxy] -2-pentenoate prepared by the process as described in Synthesis Example 2 was hydrolyzed in methanol using potassium hydroxide and water to obtain 4- [4-(4-cyanophenoxy)phenoxy] -2-pentenoic acid which was then chlorinated with thionyl chloride to obtain 4-[4-(4-cyanophenoxy)phenoxy] -2-pentenoic chloride.

1.5 g of the thus prepared 4-[4-(4-cyanophenoxy) phenoxy]-2-pentenoic chloride was dissolved in 20 ml of water-free ether, and added with 0.5 g of triethylamine and then with 0.34 g of acetone oxime, followed by agitation at room temperature for 16 hours. 50 ml of water was added to the reaction liquid, and then the ether phase was separated. The ether phase was rinsed with a dilute aqueous solution of an alkali, with a dilute hydrochloric acid solution and then with water, and dried by the addition of magnessium sulfate anhydride. Ether was distilled off, and the residue was refined by means of column chromatography (Carrier: Silica Gel, Developer: Ethyl acetate/n-Hexane = 1/1 (v/v)) to obtain 0.84 g (Yield: 50%) of an objective product which had an index of refraction of $n_D^{25}$ = 1.5581.

Representative examples of the compound of this invention are set forth in the following Table 1.

Table 1

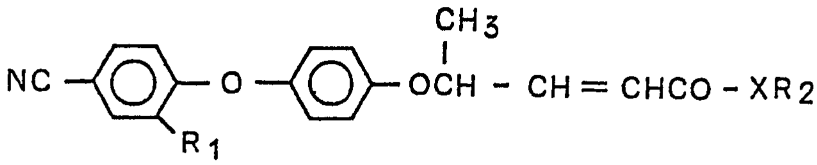

| Compound No. | $R_1$ | X | $R_2$ | Physical Constans |
|---|---|---|---|---|
| 1 | H | O | H | $n_D^{25}$ : 1.5740 |
| 2 | H | O | $Na^{\oplus} \cdot H_2O$ | mp : 194~6℃ |
| 3 | H | O | $1/2\ Ca^{++}$ | mp : 120~2℃ |
| 4 | H | O | $-CH_3$ | $n_D^{25}$ : 1.5682 |
| 5 | H | O | $-C_2H_5$ | $n_D^{25}$ : 1.5563 |
| 6 | H | O | $-C_3H_7$ (n) | $n_D^{25}$ : 1.5571 |
| 7 | H | O | $-C_3H_7$ (i) | $n_D^{25}$ : 1.5544 |
| 8 | H | O | $-C_4H_9$ (n) | $n_D^{25}$ : 1.5527 |
| 9 | H | O | $-C_8H_{17}$ (n) | $n_D^{25}$ : 1.5353 |
| 10 | Cl | O | $-C_2H_5$ | $n_D^{25}$ : 1.5682 |
| 11 | Br | O | $-C_2H_5$ | $n_D^{25}$ : 1.5774 |
| 12 | H | O | $-CH_2CF_3$ | $n_D^{25}$ : 1.5340 |
| 13 | H | O | $-CH_2-$ 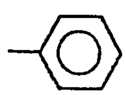 | $n_D^{25}$ : 1.5825 |
| 14 | H | S | $-CH_2CH_3$ | $n_D^{25}$ : 1.5870 |
| 15 | H | S | $-CH_2-$ 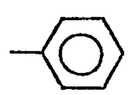 | $n_D^{25}$ : 1.6102 |
| 16 | H | O | 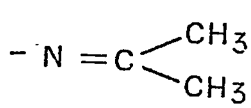 | $n_D^{25}$ : 1.5581 |

The compounds of this invention may be used in herbicide compositions by mixing appropriate quantities of one or more of the compounds represented by the general formula [I] with an inactive carrier to prepare the compositions of emulsion, hydrated form, powder form or granulated form silimar to general agricultural chemicals. Examples of solid carriers used for this purpose include talc, clay, diatomaceous earth and bentonite, and examples of liquid carriers include water, alcohols, benzene, xylene, kerosene, cyclohexane, dimethylformamide and mineral oils. In preparation of the herbicide compositions, a surface active agent or a stabilizer may be added, if necessary.

The thus prepared herbicide compositions of emulsion or hydrated form may be diluted with water to prepare suspensions or emulsions which are scattered over the surface of a field or mixed with the soil of the field before or just after the germentation of weeds. Alternatively, the suspensions or emulsions of the herbicide compositions may be scattered over the field to be spread on the stems or leaves of the weeds after they have sprouted. The herbicide compositions of powder or granulated form may be scattered over the surface of the field or mixed with the soil of the field before or just after the germentation of weeds.

The herbicide composition containing one or more compounds of this invention may be mixed with the known germicide, insecticides, mitecides, other herbicides or

agents for controlling the growth of plants.

It is sometimes preferred to mix the compound of this invention with another herbicide in order to reduce the labors or to prepare a composition which is effective to kill increased spacies of weeds. Examples of the herbicides which may be mixed and used with the compound of this invention will be listed as follows:

2,4-dichlorophenoxyacetic acid, and salts, ester and alkylamine salts thereof;

2-methyl-4-chlorophenoxyacetic acid, and salts and esters thereof;

2-methyl-4-chlorophenoxybutylic acid, and salts and esters thereof;

d,ℓ -2-(4-chloro-ortho-tolyloxy)propionic acid, and salts and esters thereof;

4-cyano-2,6-diiodophenyl octanoate;

2,4-dichlorophenyl-4'-nitrophenyl ether;

2,4,6-trichlorophenyl-4'-nitrophenyl ether;

2,4-dichlorophenyl-3'-methoxy-4'-nitrophenyl ether;

methyl 3,4-dichlorocarbanilate:

isopropyl 3-chlorocarbanilate:

S-4-chlorobenzyl diethylthiocarbamidate;

4-nitrophenyl-3',5'-xylyl ether;

S-ethyl hexahydro-1H-azepin-1-carbothioate;

3,4-dichloropropionanilide;

2-chloro-2',6'-diethyl-N-(butoxymethyl ) acetanilide;

2-chloro-2',6'-diethyl-N-(m-propoxyethyl)acetanilide;

1(α,α-dimethylbenzyl)-3-p-tolylurea;

2,4-bis(ethylamino)-6-methylthio-1,3,5-triazine;

2-ethylamino-4-isopropylamino-6-methylthio-1,3,5-triazine;

2,4-bis(isopropylamino)-6-methylthio-1,3,5-triazine;

5-tert-butyl-3-(2,4-dichloro-5-isopropoxyphenyl)-1,3,4-oxadiazoline-2-on;

2,6-dichlorothiobenzonitrile;

2,6-dichlorothiobenzamide;

2-amino-3-chloro-1,4-naphtoquinone;

2,4-dichlorophenyl-3'-carbomethoxy-4'-nitrophenyl ether;

N-p-chlorobenzyloxyphenyl-3,4,5,6-tetrahydrophthalimide;

2,4-dichlorophenyl-3'-ethoxyethoxyethoxy-4'-nitrophenyl ether;

N-(1-ethylpropyl)-2,6-dinitro-3,4-xylidine;

4-(2,4-dichlorobenzoyl)-1,3-dimethyl-pyrazol-5-yl-p-toluenesulfonate;

4(2,4-dichlorobenzoyl)-1,3-dimethyl-5-(benzolylmethoxy) pyrazole;

O,O-diisopropyl-2-(benzenesulfonamide)ethylenedithio-phosphate;

3,3'-dimethyl-4-methoxybenzophenone;

α-(2-naphtoxy)propionanilide;

O-ethyl-O-(3-methyl-6-nitrophenyl)-N-sec-butylphospho-thioamidate;

3-isopropyl-2,1,3-benzothiaziazinone-(4)-2,2-dioxide and salts thereof;

S-(2-methyl-1-piperidyl-carbonylmethyl-O,O-di-n-propyl-dithiophosphate; and

S-benzyl-N,N-dimethylthiocarbamate.

The compound of this invention may be mixed with one or more known herbicides as listed above to prepare a mixed herbicide composition which is effective to kill a variety of weeds.

The examples of the agricultural preparations of the herbicide compositions containing the compounds of this invention will be set forth below. However, it should be noted here that the agricultural preparations containing the compounds of this invention are not limited only to the exemplified preparations set forth below. "Parts" appearing in the following examples stand for "parts by weight".

Preparation Example 1:

Preparation of Hydrated Form

20 parts of Compound No. 3 prepared in accordance with the present invention is mixed with 35 parts of diatomaceous earth, 40 parts of talc, 3 parts of sodium ligninsulfonate and 2 parts of sodium dodecylbenzenesulfonate, and then pulverized, followed by the addition of water, to prepare a hydrated composition.

Preparation Example 2:

Preparation of Emulsion Form

20 parts of Compound No. 4 prepared in accordance with the present invention, 40 parts of dimethylformamide, 30 parts of xylene and 10 parts of polyoxyethylenephenyl ether are emulsified uniformly to prepare a composition of emulsion form.

Preparation Example 3:

Preparation of Granule Form

5 parts of Compound No. 7 prepared in accordance with the present invention, 15 parts of bentonite, 52.5 parts of talc, 0.5 part of sodium dodecylbenzenesulfonate, 2 parts of sodium ligninsulfonate and 25 parts of clay are mixed together and pulverized, added with water, and then extruded through a granulator to form granules which are dried and sieved to obtain a composition of granule form.

The herbicide function of the compound of this invention will be described with reference to test examples.

Test Example 1:

Soil Treating Test

20 seeds of each of wheat, soybean, Japanese radish, barnyard grass, Setalia viridis Beaur, and crabgrass were sowed on a soil of a field put in a 250 $cm^2$ pot, and covered with the soil. An emulsion of each of the tested compounds was diluted with water to prepare an emulsion containing a predetermined concentration of the compound, and the emulsion was scattered over the soil by spraying the same uniformly in a sparayed rate of 10 liters/acre. The weight of each plant which had been growing after 20 days from the date of treatment was weighed and the percentage of living grasses was calculated by comparing with the weight of grasses which had been growing in a control pot which had not been treated with the herbicide composition.

The results are shown in Table 2.

Table 2

| Compound No. | Dosed Amount of Effective Compound (g/acre) | Percentage of Living Grasses % | | | | | |
|---|---|---|---|---|---|---|---|
| | | Barnyard grass | Setalia viridis Beaur | Crabgrass | Soybean | Japanese radish | Wheat |
| 4 | 20 | 0 | 0 | 0 | 100 | 100 | 100 |
| | 10 | 0 | 0 | 0 | 100 | 100 | 100 |
| 5 | 20 | 0 | 0 | 0 | 100 | 100 | 100 |
| | 10 | 0 | 0 | 0 | 100 | 100 | 100 |
| 6 | 20 | 0 | 0 | 0 | 100 | 100 | 100 |
| | 10 | 0 | 0 | 0 | 100 | 100 | 100 |
| 7 | 20 | 0 | 0 | 0 | 100 | 100 | 100 |
| | 10 | 0 | 0 | 0 | 100 | 100 | 100 |
| 8 | 20 | 0 | 0 | 0 | 100 | 100 | 100 |
| | 10 | 0 | 0 | 0 | 100 | 100 | 100 |
| 10 | 20 | 0 | 0 | 0 | 100 | 100 | 100 |
| | 10 | 0 | 0 | 0 | 100 | 100 | 100 |
| 11 | 20 | 0 | 0 | 0 | 100 | 100 | 100 |
| | 10 | 0 | 0 | 0 | 100 | 100 | 100 |
| 12 | 20 | 0 | 0 | 0 | 100 | 100 | 100 |
| | 10 | 0 | 0 | 0 | 100 | 100 | 100 |
| 13 | 20 | 0 | 0 | 0 | 100 | 100 | 100 |
| | 10 | 0 | 0 | 0 | 100 | 100 | 100 |
| 14 | 20 | 0 | 0 | 0 | 100 | 100 | 100 |
| | 10 | 0 | 0 | 0 | 100 | 100 | 100 |
| 15 | 20 | 0 | 0 | 0 | 100 | 100 | 100 |
| | 10 | 0 | 0 | 0 | 100 | 100 | 100 |
| 16 | 20 | 0 | 0 | 0 | 100 | 100 | 100 |
| | 10 | 0 | 0 | 0 | 100 | 100 | 100 |
| Untreated | - | 100 | 100 | 100 | 100 | 100 | 100 |
| | - | 100 | 100 | 100 | 100 | 100 | 100 |

Test Example 2:

Effect of Inhibiting Growth of Barnyard Grass When Rice Plant Seeds Are Sowed in Field Filled with Water

A 1/5000 are Wagner's pot was filled with a soil of a paddy field which was raked smooth, and then sowed with 20 seeds of a rice plant (a variety referred to as NIHONBARE) and 50 seeds of barnyard grass.

The depth of water filled over the soil was set to 3 centimeters at the time when the seeds of the rice plant and the seeds of barnyard grass spouted, and each of the compounds set forth in Table 3 was spread uniformly over the surface of water in the form of a diluted emulsion prepared by diluting an emulsion prepared in accordance with Preparation Example 2 with water.

After 14 days from the dosage of each herbicide composition, the herbicide effect and the harmful secondary effect on the rice plant were checked to find the results shown in Table 3.

In the following Table 3, the weight of each of the rice plant and barnyard grass which had been growing after 14 days from the date of treatment was weighed and the percentage of living grasses was calculated by comparing with the weight of grasses which has been growing in a control pot which had not been treated with the herbicide composition.

Table 3

| Compound No. | Dosed Amount of Effective Compound (g/acre) | Herbicide Effect | Harmful Secondary Effect on Rice Plant |
|---|---|---|---|
| | | Barnyard grass | Rice Plant |
| 1 | 10<br>2.5 | 0<br>73 | 100<br>100 |
| 2 | 10<br>2.5 | 0<br>91 | 100<br>100 |
| 3 | 10<br>2.5 | 0<br>55 | 100<br>100 |
| 4 | 10<br>2.5 | 0<br>0 | 100<br>100 |
| 5 | 10<br>2.5 | 0<br>0 | 100<br>100 |
| 6 | 10<br>2.5 | 0<br>0 | 100<br>100 |
| 7 | 10<br>2.5 | 0<br>0 | 100<br>100 |
| 8 | 10<br>2.5 | 0<br>0 | 100<br>100 |
| 9 | 10<br>2.5 | 0<br>73 | 100<br>100 |
| 10 | 10<br>2.5 | 0<br>0 | 100<br>100 |
| 11 | 10<br>2.5 | 0<br>0 | 100<br>100 |
| 13 | 10<br>2.5 | 0<br>0 | 100<br>100 |
| 15 | 10<br>2.5 | 0<br>0 | 100<br>100 |
| 16 | 10<br>2.5 | 0<br>0 | 100<br>100 |
| Un-treated | -<br>- | 100<br>100 | 100<br>100 |

<u>WHAT IS CLAIMED IS:</u>

1. A cyanophenoxy-phenoxypentenoic acid derivative represented by the general formula of:

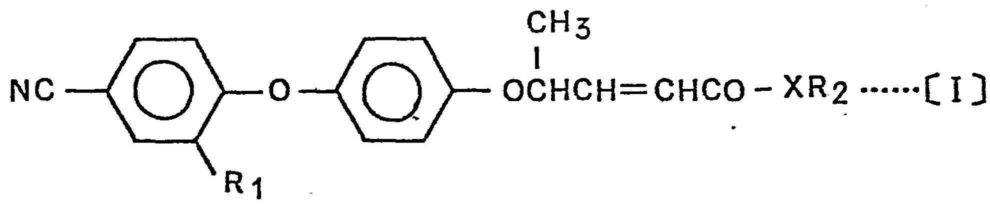

wherein $R_1$ is hydrogen, chlorine or bromine atom, X is oxygen or sulfur atom, and $R_2$ is hydrogen atom, cation, alkyl,fluoroalkyl, benzyl or -N=C(dialkyl) group.

2. A process for the preparation of a cyanophenoxy-phenoxypentenoic acid derivative represented by the general formula of:

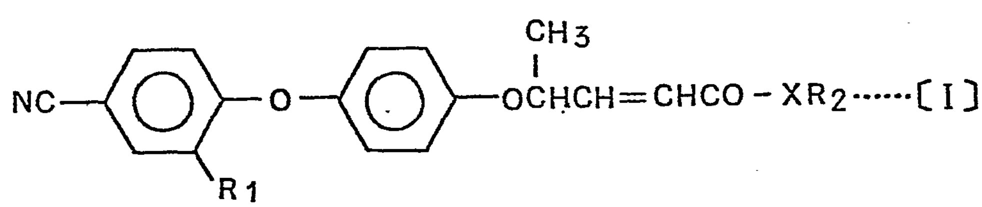

wherein $R_1$ is hydrogen, chlorine or bromine atom, X is oxygen or sulfur atom, and $R_2$ is hydrogen atom, cation, alkyl, fluoroalkyl, benzyl or -N=C(dialkyl) group;

comprising the step of reacting, in the presence of a

base, a cyanophenoxy-phenol derivative represented by the general formula of:

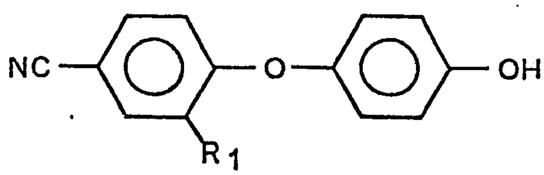

······················ [II]

wherein $R_1$ is hydrogen, chlorine or bromine atom; with a 4-halocarboxylic acid derivative represented by the general formula of:

$$\mathrm{Hal\overset{CH_3}{\overset{|}{C}}H - A - COXR_2}$$ ······················[III]

wherein A is $-\underset{Hal}{CH}-CH_2$ or -CH=CH-, Hal is a halogen atom, X is oxygen or sulfur atom, $R_2$ is hydrogen atom, cation, alkyl, fluoroalkyl, benzyl or -N=C(dialkyl) group.

3. A selective herbicide composition essentially containing, as the effective component, a cyanophenoxy-phenoxypentenoic acid derivative represented by the general formula of:

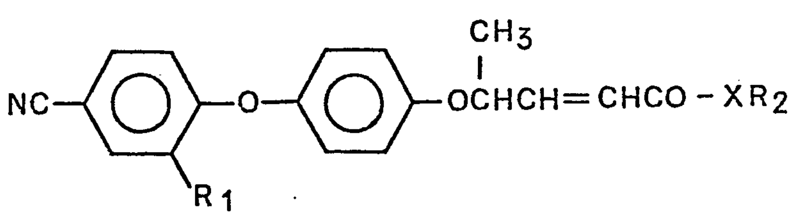

··· [I]

wherein $R_1$ is hydrogen, chlorine or bromine atom, X is oxygen or sulfur atom, and $R_2$ is hydrogen atom, cation, alkyl, fluoroalkyl, benzyl or -N=C(dialkyl) group.

0101094

European Patent Office

# EUROPEAN SEARCH REPORT

Application number

EP 83108089.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | FR - A1 - 2 440 352 (IHARA CHEMICAL INDUSTRY CO., LTD.)<br>* Claim 1; example 15; page 1, lines 1-3 *<br>-- | 1-3 | C 07 C 121/75<br>C 07 C 153/023<br>C 07 C 153/11<br>A 01 N 39/02 |
| A | US - A - 4 263 040 (K. JIKIHARA et al.)<br>* Claim 1; column 3; preparations 1-1, 2-1, 4-1, 5-1, 6-1 and 7-1 *<br>---- | 1-3 | |
| | | | TECHNICAL FIELDS SEARCHED (Int. Cl. 3) |
| | | | C 07 C 121/00<br>C 07 C 153/00<br>C 07 C 59/00<br>C 07 C 69/00 |

The present search report has been drawn up for all claims

| Place of search | Date of completion of the search | Examiner |
|---|---|---|
| VIENNA | 28-10-1983 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82